Europäisches Patentamt

(19) European Patent Office

Office européen des brevets

(11) EP 0 517 571 B1

(12) FASCICULE DE BREVET EUROPEEN

(45) Date de publication et mention
de la délivrance du brevet:
**31.01.1996 Bulletin 1996/05**

(51) Int. Cl.⁶: **C07C 67/08**, C07C 69/68

(21) Numéro de dépôt: **92401485.5**

(22) Date de dépôt: **01.06.1992**

(54) **Procédé pour la préparation d'esters d'acides racémisables**

Verfahren zur Herstellung von Estern von racemierbaren Säuren

Process for the preparation of esters of racemizable acids

(84) Etats contractants désignés:
**BE CH DE FR GB IT LI NL**

(30) Priorité: **05.06.1991 FR 9106786**

(43) Date de publication de la demande:
**09.12.1992 Bulletin 1992/50**

(73) Titulaire: **RHONE-POULENC CHIMIE**
**F-92408 Courbevoie Cédex (FR)**

(72) Inventeur: **Alas, Michel**
**F-79500 Melle (FR)**

(74) Mandataire: **Dutruc-Rosset, Marie-Claude et al**
**F-92408 Courbevoie Cédex (FR)**

(56) Documents cités:
**EP-A- 0 287 426**       **DE-A- 3 222 837**
**US-A- 2 565 487**

Printed by Rank Xerox (UK) Business Services
2.9.7/3.4

## Description

La présente invention concerne notamment un procédé pour la préparation d'esters. Elle a plus particulièrement pour objet un procédé d'estérification de sel d'acides facilement isomérisables, ledit sel présentant comme cation des ions choisis parmi les ammoniums ou mélanges d'ammoniums dont la mine associée est volatile. Ce procédé est particulièrement bien adapté à l'estérification des carboxylates optiquement actifs.

Il est fréquent, parmi les produits à activité biologique présentant un carbone asymétrique, qu'un seul diastéréoisomère soit réellement actif.
Dans les mélanges racémiques, l'autre isomère devient un élément inerte, voire cause de pollution. Il est donc souhaitable d'effectuer une synthèse asymétrique souvent par voie biologique.

Une fois le carbone asymétrique créé, souvent par une étape de fermentation, et lorsque ce carbone asymétrique est porté par un acide, l'ester est en général un intermédiaire quasi obligé pour les étapes ultérieures de la synthèse.

Sur un plan industriel, lors des fermentations conduisant à des acides asymétriques, pour avoir un procédé aussi peu polluant que possible, il serait particulièrement intéressant de réaliser la régulation du pH par l'ammoniac ou l'ammoniaque, si à l'occasion de l'estérification, il était possible de récupérer au moins en partie l'ammoniac utilisé.

Toutefois on ne dispose pas de procédé permettant une synthèse des esters à partir des sels d'ammonium qui soit rapide et/ou qui respecte la pureté isomérique, en général optique, des acides.

Dans la suite de la description, on se référera aux acides lactiques qui constituent un bon exemple des problèmes rencontrés lors de l'estérification des acides facilement isomérisables.

En d'autres termes, l'acide lactique servira de paradigme des acides facilement isomérisables. Il constitue également un acide particulièrement bien adapté aux techniques spécifiques ci-après.

L'estérification des lactates d'ammonium racémiques a fait l'objet de nombreuses publications et titres de propriété industrielle.

Ainsi, E. FILACHIONE et al, dans son brevet U.S 2.565.487 du 27 Décembre 1948, décrit l'estérification par des alcools lourds (nombre de carbone > 4) de mélanges racémiques de lactates d'ammonium conduisant à de faibles rendements en esters racémiques (entre 17 et 65 % suivant l'ester) malgré l'utilisation possible de catalyseurs, l'élimination continuelle de l'eau et l'ammoniac formés et le recyclage de l'alcool.

Un peu plus tard, ces travaux ont été repris sans améliorer fondamentalement les résultats. En effet, E. FILACHIONE dans IEC 44(9) 2189-91 en 1952 a effectué cette même réaction à température élevée (150°C) et en recyclant les intermédiaires. Il a pu obtenir un rendement de 85 % en ester butylique.

Pour améliorer les rendements de cette estérification, des auteurs se sont tournés vers l'addition d'acide ajouté comme catalyseur au départ avec le sel d'acide lactique. L'effet bénéfique enregistré n'est véritablement sensible qu'avec au moins 0,1 à 0,5 équivalent d'acide par mole de lactate, (généralement de l'acide sulfurique). On engendre alors des quantités importantes de sulfate qu'il faut éliminer et qui ne sont pas solubles dans le mélange réactionnel, lors de l'estérification.

Au cours de la présente étude, il a été montré que lorsque l'on part d'un acide optiquement pur, tous ces procédés induisent une racémisation importante du motif lactique de départ. Ainsi, ces techniques ne peuvent pas être utilisées telles quelles pour l'estérification d'isomères purs ou présentant un excès d'un isomère par rapport à l'équilibre thermodynamique.
C'est pourquoi, un but de la présente invention est de fournir un procédé perfectionné pour la préparation d'ester, par réaction d'un carboxylate d'ammonium sur un alcool, avec un rendement accru de transformation en ester, ce qui est économiquement primordial.

Un autre but de l'invention est de réaliser l'estérification tout en conservant la pureté isomérique du groupe carboxylate de départ, donc, en empêchant la racémisation de ce motif.

Un autre but enfin de la présente invention est de fournir un procédé qui permette l'amélioration de la cinétique d'estérification et ce, même à des températures relativement basses.

Ces buts et d'autres qui apparaîtront par la suite, sont atteints au moyen d'un procédé de préparation d'ester comportant une étape a) d'estérification d'un carboxylate d'ammonium optiquement actif par un alcool, où le milieu réactionnel est soumis à une pression réduite, de préférence à une pression réduite de $10^3$ Pascal à $10^5$ Pascal.

La pression est choisie dans l'intervalle précité.
La limite supérieure n'est pas comprise.
La limite inférieure n'est pas critique. Toutefois, elle est déterminée de telle sorte qu'elle soit supérieure ou égale à la pression qui provoquerait, à la température de réaction choisie, l'ébullition du mélange réactionnel qui comprend l'alcool mis en oeuvre et l'ester formé.

La pression réduite est choisie avantageusement entre $10^3$ Pascal et $5 \times 10^4$ Pascal.

Ainsi, il a été montré que, le fait de travailler à basse température et sous pression réduite permettait de réduire, de manière significative, le phénomène d'isomérisation.

La température optimale est en générale comprise entre 90 et 120°C, de préférence entre 90 à 100°C ; dans la présente description, sauf lorsque cela est spécifié autrement, les zéros de position ne sont pas significatifs.

Selon le procédé de l'invention, on fait réagir le carboxylate d'ammonium et l'alcool, à basse température et sous pression réduite.

La quantité d'alcool à mettre en jeu, exprimée par rapport au carboxylate d'ammonium, est au moins égale à la quantité stoechiométrique, de préférence en excès pouvant atteindre 1000 % et représentant plus particulièrement de 500 à 600 % de la quantité stoechiométrique.

Le temps de séjour des différents réactifs avec leur proportion respective au sein du milieu réactionnel est avantageusement de 8 à 48 heures, de préférence de 10 à 30 heures.

Selon la présente invention, on préfère pour les estérifications, utiliser des alcools secondaires ou primaires, de préférence ces derniers.

En effet, la racémisation est d'autant plus importante que l'alcool est difficile à estérifier, en outre lorsque les acides à estérifier sont des acides-alcools, il convient de tenir compte de l'existence d'une réaction parasite d'estérification entre la fonction acide et la fonction alcool de deux acides alcools pour former des polyesters. Dans le cas de l'acide lactique, la fonction alcool est une fonction secondaire ce qui est susceptible de rendre délicate l'estérification d'alcool secondaire.

Les alcools les plus couramment utilisés pour l'estérification sont les monoalcools présentant de 1 à 5 atomes de carbone.

Il est toutefois possible de réaliser des estérifications selon la présente invention avec des molécules plus lourdes pouvant avoir jusqu'à 20 atomes de carbone. Dans ce cas, il convient d'adapter les conditions de pression et d'apport d'un tiers solvant ayant un point d'ébullition convenable. Ce solvant sera de préférence un hydrocarbure aliphatique, cycloaliphatique ou aromatique dont le point d'ébullition sera compris entre 50 et 150°C sous la pression atmosphérique. A titre d'exemples, on peut mentionner notamment l'heptane, le cyclohexane, le toluène, le xylène.

Les acides présentant une fonction acide et une fonction basique sont mal adaptés au procédé de la présente invention, ce qui élimine les acides aminés.

Les acides donnant les meilleurs résultats avec la présente invention sont les acides optiquement actifs, notamment ceux ayant le carbone asymétrique en alpha d'une fonction électroattractrice comme c'est le cas de l'acide lactique, des acides halogéno-2-propioniques.

Le procédé selon la présente invention, comporte avantageusement une étape de récupération de l'ammoniac au fur et à mesure de son passage en phase vapeur.

L'ammoniac, ainsi récupéré, est en général récupéré par absorption à température convenable dans une phase aqueuse.

On ne s'écartera pas de l'invention en utilisant, en lieu et place de l'ion ammonium ($NH_4^+$) et de l'ammoniac ($NH_3$), des ammoniums substitués et les amines correspondantes à condition que lesdites amines soient volatiles (c'est-à-dire dont le point d'ébullition est inférieur à 100°C sous $10^5$ Pascal) et qu'elles présentent une bonne affinité pour l'eau. On peut citer notamment, les amines primaires, secondaires ou tertiaires ayant un nombre de carbone au plus égal à 6.

L'eau formée au cours de l'étape d'estérification ainsi que celle éventuellement introduite avec le carboxylate d'ammonium est éliminée par passage à l'état de vapeur notamment par formation d'azéotrope, de préférence, d'hétéroazéotrope.

Les espèces volatilisées telles que l'ammoniac, l'eau et éventuellement l'alcool et/ou un diluant, sont séparées les unes des autres afin de permettre le recyclage de l'alcool et de l'éventuel diluant, l'élimination de l'eau et la récupération de l'ammoniac.

Ladite séparation est avantageusement réalisée par distillation.

Le présent procédé convient particulièrement bien aux solutions concentrées d'acides provenant de fermentation. Une fois la réaction d'estérification terminée, le mélange réactionnel est avantageusement soumis à une distillation qui permettra de récupérer, d'une part, l'alcool utilisé et, d'autre part, un culot formé essentiellement de l'ester recherché, des polyesters et des impuretés introduites en même temps que les réactifs.

La distillation peut être poursuivie, de préférence, sous pression réduite (vide primaire ou de préférence secondaire), permettant de récupérer une tête de distillation formée essentiellement de l'ester recherché et un culot formé des impuretés et de polyesters.

Il est également possible de récupérer par distillation, les éventuels diluants évoqués ci-dessus.

L'alcool récupéré au cours de ces diverses opérations de distillation, est avantageusement recyclé, ainsi que cela a été mentionné auparavant, vers l'étape d'estérification.

Le culot issu de la dernière distillation, formé de polyesters dans le cas d'acides d'alcools, est avantageusement soumis à une lyse, de préférence à un solvolyse, au moyen dudit alcool ci-dessus ou une hydroalcoolyse au moyen d'un mélange entre l'eau et ledit alcool.

Cette opération de lyse est conduite avantageusement à une température comprise entre 100 et 130°C et pendant un temps de séjour compris entre, 30 minutes et 4 heures, avec un rapport entre le mélange eau/alcool et le résidu compris entre 0 et 30 %.

Le mélange eau/alcool contient avantageusement entre 0 et 30 % d'alcool.

Le produit de la lyse ainsi obtenu est avantageusement séparé d'avec les autres impuretés par distillation, en général sous vide.

Ce procédé est particulièrement bien adapté au traitement des solutions à une teneur de 60 à 70 % en poids de lactates d'ammonium.

Ledit lactate d'ammonium peut provenir d'une quelconque opération de fermentation connue de l'homme de métier.

Ces teneurs peuvent être obtenues simplement par concentration de moûts fermentaires.

Le procédé de l'invention est particulièrement intéressant, car il conserve la qualité isomérique de l'acide lactique de départ, à savoir, il génère moins de 5 % de préférence moins de 3 %, d'isomère L quand on part d'acide D ou inversement.

L'invention sera illustrée par les exemples suivants qui ne sont en aucun cas limitatifs de l'invention.

## EXEMPLES

### Exemple 1 : (comparatif)

Dans un réacteur ayant un volume de travail à V litres et surmonté d'une colonne équivalente à 15 plateaux théoriques, on introduit en continu 0,87 V kg/jour d'une solution aqueuse concentrée de lactate d'ammonium obtenu par concentration directe d'un moût de fermentation et titrant 75 % P/P [pureté isomérique L/(L+D) = 2 %]. On alimente en parallèle 3.1 V kg/jour de méthanol anhydre (neuf + récupéré). Le réacteur est chauffé pour maintenir une température de 115°C dans la masse. Ce qui coule du réacteur est successivement distillé sous vide 240 torr pour récupérer le méthanol puis sous vide 50 torr pour le lactate de méthyle. Les lourds restant en résidu sont recyclés directement dans le réacteur R1. Quand le régime d'équilibre de l'ensemble est atteint, la quantité de lourds n'évolue plus et l'on produit 0.60 V kg/jour de lactate de méthyle. Le lactate de méthyle obtenu à une pureté isomérique [rapport L/(L+D)] égale à 8 %, soit un taux de racémisation de plus de 12 %.

### Exemple 2 :

On opère comme dans l'exemple 1 en mettant le réacteur sous un vide de 350 torr. On alimente O.45 V kg/jour de la même solution de lactate d'ammonium de l'exemple 1 (pureté isomérique 2 %) et 2.2 V kg/jour de méthanol. La température se stabilise à 100°C. Quand le régime de l'équilibre est atteint, il n'y a plus augmentation de la quantité de lourds et l'on produit 0.31 V kg/jour de lactate de méthyle [pureté isomérique (L/(L+D)= 4.5 %) ], soit un taux de racémisation de 5 %.

### Exemple 3 :

On reconduit les conditions de l'exemple 2 en réalisant une hydroalcoolyse de lourds sous pression avant leur recyclage au réacteur (température 130°C . 10 % d'un mélange eau/méthanol par rapport aux lourds, durée du traitement 3 heures). Quand le système est stabilisé, on est capable d'alimenter O.49 V kg/jour de lactate d'ammonium en récupérant O.34 V Kg/jour de lactate de méthyle. La pureté isomérique est inchangée par rapport à l'exemple 2.

### Exemple 4 :

On opère comme dans l'exemple 1 en remplaçant le méthanol par le butanol normal. Le réacteur est mis sous vide 310 torr. Le produit coulant du réacteur est successivement rectifié dans une première colonne opérant sous 120 torr puis dans une seconde opérant sous 20 torr. En régime stabilisé, quand il n'y a plus évolution de la masse de lourds, on est capable d'alimenter O.62 V kg/jour de la solution de lactate d'ammonium (titre 75 % pureté isomérique 2 %) en tirant O.62 V kg/jour de lactate de butyle ayant une pureté isomérique égale à 3 %, soit un taux de racémisation de 2 %.

### Exemple 5

On opère comme dans l'exemple 4 en remplaçant le butanol normal par l'isobutanol. Les conditions sont strictement identiques. On obtient 0.63 V Kg/jour de lactate d'isobutyle ayant une pureté isomérique de 3.1%, (ratio L/L + D en %) en partant de 0.63 V Kg/jour de la même solution de lactate d'ammonium à 75 % en poids.

## Revendications

1. Procédé de préparation d'un ester d'acide facilement isomérisable caractérisé par le fait qu'il comporte une étape a) d'estérification d'un carboxylate d'ammonium optiquement actif par un alcool, où le milieu réactionnel est soumis à une pression réduite, de préférence, à une pression réduite de $10^3$ à $10^5$ Pascal, de préférence, de $10^3$ à $5x10^4$ Pa, limite supérieure non comprise.

2. Procédé selon la revendication 1 caractérisé par le fait que le milieu réactionnel est soumis à une température comprise entre 90 et 120°C, de préférence entre 90 et 100°C.

3. Procédé selon l'une des revendications 1 et 2 caractérisé par le fait que le temps de séjour des différents réactifs avec leur proportion respective au sein du milieu réactionnel est de 10 à 30 heures.

4. Procédé selon l'une des revendications 1 à 3, caractérisé par le fait qu'il comporte en outre une étape b) de séparation, en général par distillation, de l'ammoniac et/ou de l'amine d'avec les espèces volatilisées au cours de la reaction de l'étape a).

5. Procedé selon la revendication 4 caractérisé par le fait que ledit amoniac est récuperé après la dite étape b) de séparation et après une étape c) d'absorption dans de l'eau sous la forme d'une solution aqueuse concentrée recyclable en fermentation.

6. Procédé selon l'une quelconque des revendications 1 à 5 caractérisé par le fait que l'eau, au cours de l'étape a), éliminée sous forme de vapeur d'eau, est separée d'avec les autres espèces volatiles au cours de l'étape c. simultanée à l'étape b.

7. Procédé selon la revendication 6 caractérisé par le fait l'eau est récupérée par condensation.

8. Procédé selon l'une des revendications 1 à 7 caractérisé par le fait que ledit-alcool, volatilisé lors de l'étape a) est récupéré sous la forme d'un mélange eau-alcool au cours de l'étape b.

9. Procédé selon la revendication 8 caractérisé par le fait que l'alcool obtenu, après éventuel traitement, est recyclé vers l'étape a.

10. Procédé selon l'une des revendications 1 à 9 caractérisé par le fait que l'on soumet le mélange réactionnel issu de l'étape a) à une étape d) de distillation sous pression réduite et on récupère un mélange dudit ester et dudit alcool.

11. Procédé selon la revendication 10 caractérisé par le fait que le mélange alcool et ester est recyclé vers l'étape a).

12. Procédé selon l'une des revendication 10 et 11 caractérisé par le fait que le culot issu de d) est soumis à une distillation sous pression réduite pour donner d'une part une fraction volatile constitué essentiellement d'un ester désigné et d'autre part un résidu.

13. Procédé selon la revendication 12 caractérisé par le fait que ledit résidu est soumis à une étape de lyse f).

14. Procédé selon la revendication 13 caractérisé par le fait que ladite lyse est réalisée par une mélange d'eau et dudit-alcool (puis recyclés au sein du milieu reactionnel.).

15. Procédé selon l'une des revendications 13 et 14 caractérisé par le fait la lyse du dit-condensat est faite à une température comprise entre 100 et 130°C et pendant un temps de séjour compris entre 30 minutes et 4 heures, avec un rapport entre le mélange eau-alcool et le résidu compris entre 0 et 30 %.

16. Procédé selon l'une des revendications 1 à 15 caractérisé par le fait que le carboxylate d'ammonium est le lactate d'ammonium optiquement actif.

17. Procédé selon l'une des revendications 1 à 16 caractérisé par le fait que l'alcool est un alcool primaire ou secondaire ayant de 1 à 5 atomes de carbone, de préférence le méthanol, le butanol et l'isobutanol.

## Claims

1. A process for the preparation of a readily isomerisable ester, characterised in that it comprises a step (a) of esterification of an optically active ammonium carboxylate with an alcohol, where the reaction medium is subjected to a reduced pressure, preferably of $10^3$ Pascal to $10^5$ Pascal, more preferably $10^3$ to $5 \times 10^4$ Pascal, not including the upper limit.

2. A process according to claim 1, characterised in that the reaction medium is subjected to a temperature of between 90°C and 120°C, preferably between 90°C and 100°C.

3. A process according to claim 1 or claim 2, characterised in that the residence time for the reactants in their respective proportions in the reaction medium is in the range 10 hours to 30 hours.

4. A process according to any one of claims 1 to 3, characterised in that it further contains a step (b) for separation, in general by distillation, of ammonia and/or the amine with the species volatilised during the reaction in step (a).

5. A process according to claim 4, characterised in that the ammonium is recovered after said separation step (b) and after a step (c) for absorption in water in the form of a concentrated aqueous solution which can be recycled to the fermentation step.

6. A process according to any one of claims 1 to 5, characterised in that during step (a), the water eliminated as water vapour is separated with the other volatile species during step (c) simultaneously with step (b).

7. A process according to claim 6, characterised in that the water is recovered by condensation.

8. A process according to any one of claims 1 to 7, characterised in that said alcohol, volatilised during step (a), is recovered during step (b) in the form of a water-alcohol mixture.

9. A process according to claim 8 characterised in that the alcohol obtained is recycled to step (a) after optional treatment.

10. A process according to any one of claims 1 to 9, characterised in that the reaction mixture from step (a) is distilled under reduced pressure in a step (d) and a mixture of said ester and said alcohol is recovered.

11. A process according to claim 10, characterised in that the mixture of alcohol and ester is recycled to step (a).

12. A process according to claim 10 or claim 11, characterised in that the residue from step (d) is distilled under reduced pressure to produce a volatile fraction essentially constituted by the desired ester and by a residue.

13. A process according to claim 12, characterized in that said residue is treated in a lysing step (f).

14. A process according to claim 13, characterized in that said lysis is carried out by a mixture of water and said alcohol (then recycled to the reaction medium).

**15.** A process according to claim 13 or claim 14, characterized in that lysis of said condensate is carried out at a temperature of between 100°C and 130°C for a residence time of between 30 minutes and 4 hours, with a ratio between the water-alcohol mixture and the residue of between 0% and 30%.

**16.** A process according to any one of claims 1 to 15, characterized in that the ammonium carboxylate is optically active ammonium lactate.

**17.** A process according to any one of claims 1 to 16, characterized in that the alcohol is a primary or secondary alcohol containing 1 to 5 carbon atoms, preferably methanol, butanol or isobutanol.

**Patentansprüche**

**1.** Verfahren zur Herstellung eines Esters einer leicht isomerisierbaren Säure, dadurch gekennzeichnet, daß es einen Veresterungsschritt a) eines optisch aktiven Ammoniumcarboxylats mit einem Alkohol umfaßt, wobei das Reaktionsmilieu einem reduzierten Druck, vorzugsweise einem reduzierten Druck von $10^3$ bis $10^5$ Pa, insbesondere von $10^3$ bis $5 \cdot 10^4$ Pa, unterworfen wird, wobei die Obergrenze nicht eingeschlossen ist.

**2.** Verfahren nach Anspruch 1, dadurch gekennzeichnet, daß das Reaktionsmilieu einer Temperatur zwischen 90 und 120 °C, vorzugsweise zwischen 90 und 100 °C, unterworfen wird.

**3.** Verfahren nach einem der Ansprüche 1 und 2, dadurch gekennzeichnet, daß die Verweildauer der verschiedenen Reagenzien mit ihrer jeweiligen Menge im Reaktionsmilieu 10 bis 30 Stunden beträgt.

**4.** Verfahren nach einem der Ansprüche 1 bis 3, dadurch gekennzeichnet, daß es zusätzlich einen Abtrennschritt b), im allgemeinen durch Destillation, des Ammoniaks und/oder des Amins von den im Laufe des Reaktionsschritts a) abgedampften Substanzen umfaßt.

**5.** Verfahren nach Anspruch 4, dadurch gekennzeichnet, daß der Ammoniak nach dem Abtrennschritt b) zurückgewonnen wird und nach einem Absorptionsschritt c) in Wasser in Form einer konzentrierten, wäßrigen, für die Gärung wiederverwendbaren Lösung zurückerhalten wird.

**6.** Verfahren nach einem der Ansprüche 1 bis 5, dadurch gekennzeichnet, daß das Wasser, das im Verlauf von Schritt a) in Form von Wasserdampf entfernt wird, von den anderen flüchtigen Substanzen im Verlauf von Schritt c) zusammen mit Schritt b) abgetrennt wird.

**7.** Verfahren nach Anspruch 6, dadurch gekennzeichnet, daß das Wasser durch Kondensation zurückgewonnen wird.

**8.** Verfahren nach einem der Ansprüche 1 bis 7, dadurch gekennzeichnet, daß der Alkohol, der in Schritt a) verdampft wird, in Form einer Wasser/Alkohol-Mischung im Verlauf von Schritt b) wiedergewonnen wird.

**9.** Verfahren nach Anspruch 8, dadurch gekennzeichnet, daß der erhaltene Alkohol, gegebenenfalls nach Behandlung, Schritt a) wieder zugeführt wird.

**10.** Verfahren nach einem der Ansprüche 1 bis 9, dadurch gekennzeichnet, daß man die Reaktionsmischung aus Schritt a) einem Destillationsschritt d) unter reduziertem Druck unterwirft und man eine Mischung des Esters und des Alkohols erhält.

**11.** Verfahren nach Anspruch 10, dadurch gekennzeichnet, daß die Alkohol/Ester-Mischung wieder Schritt a) zugeführt wird.

**12.** Verfahren nach einem der Ansprüche 10 und 11, dadurch gekennzeichnet, daß der Rückstand aus Schritt d) einer Destillation unter reduziertem Druck unterworfen wird, die zum einen eine flüchtige Fraktion, die im wesentlichen aus einem bestimmten Ester zusammengesetzt ist, und zum anderen einen Rückstand liefert.

**13.** Verfahren nach Anspruch 12, dadurch gekennzeichnet, daß der Rückstand einem Löseschritt f) unterworfen wird.

**14.** Verfahren nach Anspruch 13, dadurch gekennzeichnet, daß der Löseschritt mit einer Mischung aus Wasser und besagtem Alkohol durchgeführt wird (die dann dem Reaktionsmilieu wieder zugeführt werden).

**15.** Verfahren nach einem der Ansprüche 13 und 14, dadurch gekennzeichnet, daß der Löseschritt des Kondensats bei einer Temperatur zwischen 100 und 130 °C und bei einer Verweildauer zwischen 30 Minuten und 4 Stunden und mit einem Verhältnis von Mischung Wasser/Alkohol zu Rückstand zwischen 0 und 30 % durchgeführt wird.

**16.** Verfahren nach einem der Ansprüche 1 bis 15, dadurch gekennzeichnet, daß das Ammoniumcarboxylat optisch aktives Ammoniumlactat ist.

**17.** Verfahren nach einem der Ansprüche 1 bis 16, dadurch gekennzeichnet, daß der Alkohol ein primärer oder sekundärer Alkohol mit 1 bis 5 Kohlenstoffatomen, vorzugsweise Methanol, Butanol und Isobutanol, ist.